# EUROPEAN PATENT SPECIFICATION

(11) **EP 3 593 796 B1**
(45) Date of publication and mention of the grant of the patent: **23.07.2025**
(21) Application number: 18763939.8
(22) Date of filing: 07.03.2018
(51) Int. Cl.: A61K 31/203, A61K 31/65, A61K 31/60, A61P 17/10, A61K 31/192

(54) **COMPOSITION FOR THE TOPICAL TREATMENT OF SEVERE ACNE**
ZUSAMMENSETZUNG ZUR TOPISCHEN BEHANDLUNG VON SCHWERER AKNE
COMPOSITION POUR LE TRAITEMENT TOPIQUE DE L'ACNÉ SÉVÈRE

(30) Priority: 08.03.2017 MX 2017003003
(43) Date of publication of application: 15.01.2020
(73) Proprietor: Centro Internacional de Cosmiatría, S.A.P.I. de C.V., 01030 Ciudad De México (MX)
(72) Inventor: NACHT, Sergio, Las Vegas, Nevada 89135 (US)
(74) Representative: Elzaburu S.L.P.
(86) International application number: PCT/MX2018/000017
(87) International publication number: WO 2018/164566

(56) References cited:
- WO-A2-2007/065289
- WO-A2-2008/051461
- ES-T3- 2 423 951
- RU-C1- 2 500 395
- US-A- 4 487 782
- US-A- 5 643 949
- US-A1- 2006 177 392
- US-A1- 2016 338 985
- THIELITZ ANJA ET AL: "Topical retinoids in acne vulgaris: update on efficacy and safety.", AMERICAN JOURNAL OF CLINICAL DERMATOLOGY 2008, vol. 9, no. 6, 2008, pages 369 - 381, XP009523184, ISSN: 1175-0561
- THIELITZ A ET AL: "Topical retinoids in acne - An evidence-based overview", JDDG - JOURNAL OF THE GERMAN SOCIETY OF DERMATOLOGY 200812 GB, vol. 6, no. 12, December 2008 (2008-12-01), pages 1023 - 1031, XP009523195, ISSN: 1610-0379

## Description

### Background of the invention

### Acne Vulgaris Pathogenic Factors

As known, for acne to occur there must be three abnormal factors simultaneously:
A high secretion of the sebaceous gland, an abnormal keratinization of the walls of the pilosebaceous follicle and the presence of a facultative anaerobic bacterium, *Propionibacterium acnes (P.acnes),* that multiplies in the sebaceous gland feeding on triglycerides and releasing fatty acids that generate an inflammatory process.

### Therapeutic Strategies

All effective topical therapies are aimed at controlling one or more of these factors, as this results in an improvement of the acne condition.

Reduction in sebaceous secretion: The sebaceous gland is under hormonal control, particularly androgens and especially testosterone. Therefore, a reduction in androgen secretion reduces the activity of the sebaceous gland. Systemic estrogen administration has this effect, but, for obvious reasons, this type of medication is only acceptable for treating women since in men it has unacceptable feminizing side effects.

Oral administration of 13-cis-retinoic acid (Accutane in the US) has a profound effect on the sebaceous gland since it almost completely suppresses this secretion and thus causes a marked improvement in almost 100% of patients.

However, the side effects are severe and range from severe dryness of the skin, lips and even eyes to acute teratogenic effects in pregnant women. This makes this therapy reserved for very severe acne (conglobata) and in cases where other therapeutic forms have not worked. In addition, this therapy requires the simultaneous use of two forms of contraceptives to prevent pregnancy, which is not acceptable for many women for religious or psychological reasons.

Normalization of keratinization of the follicular wall: There are therapeutic agents that lead to this result, from Salicylic Acid, Sulfur (with or without Resorcinol)and retinoids such as all-*trans*-retinoic Acid, Adapalene, Tazarotene or Retinol.

Benzoyl Peroxide also has a minor keratolytic action.

Reduction of bacterial flora: This can be obtained either by oral or topical administration of antibiotics or by topical administration of some bactericidal compounds. However, it is important that the active compounds have a significant solubility (or partition coefficient) in the sebaceous secretion since that is where *P. acnes* dwells and multiplies. That is why tetracyclines are the antibiotics of choice (oral or topical) and Benzoyl Peroxide is the most effective topical bactericide.

In the World Dermatological Market there is a large number of topical products for the treatment of acne that act on one or two of the pathogenic factors of this condition. That is, they treat either the abnormal keratinization of the pilosebaceous follicle, the bacterial proliferation or both, but they have no effect on sebaceous secretion which is the initial and most important factor in this process since this secretion is what results in the adhesion of the Pilosebaceous duct wall cells preventing their normal desquamation. This results in the "plugging" of the duct generating comedones (open or closed) that are the initial acne lesion. These comedones provide a favorable field for the development of *P.acnes,* since this is a facultatively anaerobic bacterium, which proliferates in the closed follicle and initiates the inflammatory process. Document: THIELITZ ANJA ET AL: "Topical retinoids in acne vulgaris: update on efficacy and safety.", AMERICAN JOURNAL OF CLINICAL DERMATOLOGY 2008, vol. 9, no. 6, 2008, pages 369-381, XP9523184,ISSN: 1175-0561discloses combination therapy with either tretinoin or isotretinoin and antibiotics such as clindamycin for treating acne.

### Detailed Description of the Invention

The invention described herein can provide for the first time an effective and simultaneous topical treatment against all THREE PATHOGENIC FACTORS of acne.

These compositions result in high effectiveness with virtually no side effects and, since the application of 13-cis-retinoic acid (isotretinoin) is only topical and not systemic, this avoids possible teratogenic effects and significantly reduces the excessive dryness of mucous membranes and skin tissues.

### Description of the Invention

The invention consists of a topical formulation for the treatment of acne, in particular the most severe type of inflammatory acne.

The formulation contains 13-*cis*-retinoic acid in concentrations from 0.001% to 0.25% (preferably between 0.005% to 0.15%) and another retinoid with keratolytic therapeutic action in acne, namely *all-trans* retinoic acid in concentrations from 0.001% to 0.25% (preferably between 0.025% to 0.10%) and clindamycin in concentrations from 0.5% to 3%, in a cosmetically acceptable aqueous vehicle, containing suitable antioxidants and preservatives to protect retinoids against oxidation.

The formulations described in the previous paragraph contains an antibiotic capable of controlling *P. acnes,* namely clindamycin in concentrations of 0.5% to 3%, preferably between 1% and 2%. The following formulations are presently disclosed but are not part of the invention.

The formulations described in the previous paragraph in which the antibiotic used is erythromycin or some other tetracycline in concentrations between 1% and 5%.

The formulations in the previous paragraph in which the antibiotic is replaced by Benzoyl Peroxide in concentrations between 2.5% and 10%.

The formulations of the preceding paragraphs in which all-trans-retinoic acid is substituted by Adapalene or Tazarotene in concentrations between 0.001% and 0.5%, preferably between 0.025% and 0.3%.

The formulations according to the present invention and as claimed, which are described in the present description, can have one or more of the active ingredients included in polymeric particles such as those described in US Patent No. 5, 145, 675 and in US Patent No. 9, 149, 490 to obtain a gradual and extended release of the active principle (s) included in the polymer system: said polymer particles are mainly composed of crosslinked polystyrene or polymethacrylates, more commonly divinylbenzene for the polystyrene polymer and ethylene glycol dimethacrylate for polymethacrylates. The polymeric particles have a particle size in the range of 5 to 300 µm, more commonly in the range of 10 to 25 µm; and a pore diameter and volume between 0.3 to 4 cm³/g, more commonly 2.0 cm³/g. This results in superior therapeutic effects with a total reduction in many cases in the side effects, especially topical irritation. This also allows reducing the dosage since in many cases a daily application is sufficient.

The formulations of the present invention for the topical treatment of acne, especially severe inflammatory acne with topical formulations contain 13-cis-retinoic acid, and another retinoid with keratolytic action which is all-trans-retinoic acid, and clindamycin.

One or more of the active ingredients may be included in a polymeric system suitable for obtaining a slow and extended release resulting in high efficacy with less skin irritation.

### Formulations

### Formula 1

| | |
|---|---|
| Tretinoin USP ^{1,2} | 0.12 % |
| Isotretinoin ¹ | 0.12 % |
| Clindamycin ³ | 1.20 % |
| Carbomer 934P, NF | 0.80 % |
| Glycerin USP | 12.50 % |
| Propylene glycol | 4.60 % |
| PPG-20 Methylglucose ether distearate | 4.75 % |
| Cyclomethicone and Dimethicone Copoliol | 2.30 % |
| Crosslinked Acrylate Polymer (Microsponge) | 8.85 % |
| Trolamine NF | 0.40 % |
| BHT | 0.02 % |
| Disodium edetate | 0.01 % |
| Benzyl alcohol | 0.90 % |
| Deionized water | 63.48 % |

### Notes:

1. Tretinoin and isotretinoin can be dispersed in the vehicle or pre-incorporated into the acrylate polymer
2. Tretinoin can be substituted for adapalene or tazarotene, or salicylic acid
3. Clindamycin may be substituted for erythromycin or tetracycline

### Control formulas

Formula 2: Identical to Formula 1 but without isotretinoin
Formula 3: Identical to Formula 1 but without tretinoin
Formula 4: Identical to Formula 1 but without the antibiotic

### Alternative formulas in double chamber containers:

All Retinoids are unstable in the presence of oxidizing agents such as Benzoyl Peroxide or acids such as Glycolic Acid or Salicylic Acid.

However, these compounds can be administered together with retinoids as long as they are kept separate until they are dispensed on the skin of the acne affected area.

There are new double packages (double chamber) in which the formulation is packed containing the retinoids in one chamber and the other containing the oxidizing agent or acid in the other. Each of these chambers is provided with a pump that can be operated independently or simultaneously to dispense the formulas.

The mechanical design of these containers requires that for their optimal functioning the active compounds must be formulated in vehicles of the same or very similar composition so that the formulas in each chamber have the very close to the same specific viscosity or density.

### Examples of Realization

The following formulas are examples of this type of formulations that can be used in double containers.

### Formula 5-A

### Active principles:

| | |
|---|---|
| Tretinoin USP | 0.12% |
| Isotretinoin | 0.12% |

### Vehicle

| | |
|---|---|
| Starch Phosphate | 14.40% |
| Bentonite | 7.75% |
| Zinc Oxide | 4.25% |
| Polyethylene glycol 400 | 5.00% |
| Oleth-10 | 2.20% |
| Menthol | 0.15% |
| Sodium Hexametaphosphate | 0.03% |
| Methylparaben | 0.18% |
| Propylparaben | 0.02% |
| Deionized water q.s. | 100.00% |

### Formula 5-B

### Active principles

Benzoyl Peroxide Hydrate (70%) 8.57% (instead of the antibiotic)
Note: This results in a final BPO concentration of 6.00% (nominal 5% plus 20% overload). Likewise, 4.2% BPO can be used to obtain a nominal final concentration of 2.5% or 15.5% to obtain a nominal final concentration of 10%)
Vehicle: As in Formula 5-A.

### Clinical study

The efficacy of these formulations was studied in a clinical study in which 30 acne patients between 18 and 30 years of age (12 female and 18 male) were enrolled

All of them were rated as moderate or severe acne based on the number of acne lesions present when enrolling them.

Lesions on the face and neck were counted by classifying them in **open and closed comedones** (non-inflammatory lesions) and **in Papules and Pustules** (inflammatory lesions).

Participants with at least 30 non-inflammatory lesions and 30 inflammatory lesions were selected.

Sebaceous secretion was determined in all participants in each observation using the method described by Harris, Downing, Stewart and Strauss¹

The participants were divided into five groups of 6 individuals each and assigned Formulas 1, 2, 3, 4 and 5 (5-A + 5-B). Participants were instructed to apply the assigned formula twice a day, morning and evening, after washing their face and neck with a mild assigned soap (Dove).

Sebaceous secretion and lesion count determinations were made at Time 0 (Baseline) and after 2, 4 and 8 weeks of drug use.

The data obtained are attached and tabulated as the average of each group to each observation plus or minus the standard deviation (± SD).

### Sebaceous Secretion Determination ¹

| Weeks | Formula 1 | Formula 2 | Formula 3 | Formula 4 | Formula 5 |
|---|---|---|---|---|---|
| 0 (Baseline) | 3.57 ± 1.1 | 3.65 ± 1.43 | 3.26 ± 1.11 | 3.91 ± 1.52 | 3.76 ± 1.33 |
| 2 | 2.85 ± 0.70 | 3.48 ± 1.18 | 2.96 ± 0.93 | 2.74 ± 0.97 | 2.67 ± 0.91 |
| 4 | 1.14 ± 0.16 | 3.51 ± 1.35 | 1.27 ± 0.27 | 1.19 ± 0.21 | 1.10 ± 0.20 |
| 8² | 0.35 ± 0.08 | 3.23 ± 1.21 | 0.42 ± 0.07 | 0.38 ± 0.05 | 0.43 ± 0.09 |

### Notes

1. Data are expressed as **mg/10 cm²/3 hours**
2. Formulas 1, 3, 4 and 5 vs Formula 2: p <0.01 (paired T test)

### Lesion Count

| COMEDONES (Closed + Open) | | | | | |
|---|---|---|---|---|---|
| 0 (Base) | 47 ± 11 | 39 ± 8 | 51 ± 13 | 43 ± 7 | 53± 14 |
| 2 | 30 ± 7 | 34 ± 9 | 45 ± 12 | 32 ± 8 | 43 ± 10 |
| 4 | 16 ± 5 | 23 ± 9 | 40 ± 11 | 23 ± 6 | 19± 7 |
| 8 | 7 ± 4 | 12 ± 6 | 35 ± 13 | 9 ± 5 | 8± 3 |

| PAPULES + PUSTULES | | | | | |
|---|---|---|---|---|---|
| 0 (Base) | 32 ± 13 | 36 ± 15 | 30 ± 11 | 38 ± 15 | 37 ± 16 |
| 2 | 18 ± 9 | 28 ± 13 | 27 ± 12 | 22 ± 11 | 19 ± 10 |
| 4 | 9 ± 4 | 19 ± 7 | 19 ± 8 | 17 ± 10 | 13 ± 3 |
| 8^{1,2} | 2 ± 0.5 | 13 ± 4 | 15 ± 6 | 11 ± 5 | 8 ± 0.6 |

| | | | | | |
|---|---|---|---|---|---|
| Note: All data are expressed as **Average ± SD** 1. Formulas 1 and 5 vs 2.3 and 4: p <0.01 (Student t test). 2. Formula 1 vs. 5: p <0.05 (Student t test) | | | | | |

## Claims

1. A topical formulation containing 13-*cis*-retinoic acid in concentrations from 0.001% to 0.25%, another retinoid with keratolytic therapeutic action in acne and an antibiotic capable of controlling *P. acnes* in a cosmetically acceptable aqueous vehicle containing antioxidants and preservatives suitable to protect retinoids against oxidation for use in the topical treatment of acne, wherein the other retinoid with keratolytic therapeutic action in acne is *all-trans*-retinoic acid in concentrations from 0.001% to 0.25% and wherein the antibiotic is clindamycin in concentrations from 0.5% to 3%.

2. The formulation for use according to claim 1, wherein the 13-*cis*-retinoic acid is in concentrations between 0.005% to 0.15%.

3. The formulation for use according to claim 1, wherein the *all-trans*-retinoic acid is in concentrations from 0.025% to 0.10%.

4. The formulation for use according to claim 1, wherein the clindamycin is in concentrations between 1% and 2%.

5. The formulation for use according to claim 1, wherein one or more of the active ingredients are included in polymeric particles to obtain a gradual and extended release of the active ingredient(s) included in a polymeric system.

6. The formulation for use according to claims 1 to 5, wherein the acne is severe inflammatory acne.

## Patentansprüche

1. Topische Formulierung, die 13-cis-Retinsäure in Konzentrationen von 0,001 % bis 0,25 %, ein anderes Retinoid mit keratolytischer therapeutischer Wirkung bei Akne und ein Antibiotikum enthält, das in der Lage ist, *P. acnes* in einem kosmetisch verträglichen wässrigen Trägerstoff zu kontrollieren, das Antioxidantien und Konservierungsmittel enthält, die geeignet sind, Retinoide vor Oxidation zu schützen, zur Verwendung bei der topischen Behandlung von Akne, wobei das andere Retinoid mit keratolytischer therapeutischer Wirkung bei Akne All-Trans-Retinsäure in Konzentrationen von 0,001 % bis 0,25 % ist und wobei das Antibiotikum Clindamycin in Konzentrationen von 0,5 % bis 3 % vorliegt.

2. Formulierung zur Verwendung nach Anspruch 1, wobei die 13-cis-Retinsäure in Konzentrationen zwischen
0,005 % bis 0,15 % vorliegt.

3. Formulierung zur Verwendung nach Anspruch 1, wobei die All-Trans-Retinsäure in Konzentrationen von
0,025 % bis 0,10 % vorliegt.

4. Formulierung zur Verwendung nach Anspruch 1, wobei das Clindamycin in Konzentrationen zwischen 1 % und 2 % vorliegt.

5. Formulierung zur Verwendung nach Anspruch 1, wobei einer oder mehrere der Wirkstoffe in Polymerpartikeln eingeschlossen sind, um eine allmähliche und verlängerte Freisetzung des Wirkstoffs/der Wirkstoffe zu erhalten, der/die in einem Polymersystem eingeschlossen ist/sind.

6. Formulierung zur Verwendung nach den Ansprüchen 1 bis 5, wobei die Akne schwere entzündliche Akne ist.

## Revendications

1. Formulation topique contenant de l'acide 13-cis-rétinoïque à des concentrations de 0,001 % à 0,25 %, un autre rétinoïde à action thérapeutique kératolytique dans l'acné et un antibiotique capable de contrôler *P. acnes* dans un véhicule aqueux cosmétiquement acceptable contenant des antioxydants et des conservateurs appropriés pour protéger les rétinoïdes contre l'oxydation, destinée à être utilisée dans le traitement topique de l'acné, dans laquelle l'autre rétinoïde à action thérapeutique kératolytique dans l'acné est l'acide tout-trans-rétinoïque à des concentrations de 0,001 % à 0,25 % et dans laquelle l'antibiotique est la clindamycine à des concentrations de 0,5 % à 3 %.

2. Formulation à utiliser selon la revendication 1, dans laquelle l'acide 13-cis-rétinoïque est dans des concentrations comprises entre
0,005 % à 0,15 %.

3. Formulation à utiliser selon la revendication 1, dans laquelle l'acide rétinoïque tout-trans est dans des concentrations de
0,025% à 0,10%.

4. Formulation à utiliser selon la revendication 1, dans laquelle la clindamycine est dans des concentrations comprises entre 1 % et 2 %.

5. Formulation à utiliser selon la revendication 1, dans laquelle un ou plusieurs des ingrédients actifs sont inclus dans des particules polymères pour obtenir une libération progressive et prolongée du ou des ingrédients actifs inclus dans un système polymère.

6. Formulation à utiliser selon les revendications 1 à 5, dans laquelle l'acné est une acné inflammatoire sévère.
